# EUROPÄISCHE PATENTSCHRIFT

(11) **EP 0 734 411 B1**
(45) Veröffentlichungstag und Bekanntmachung des Hinweises auf die Patenterteilung: **14.04.1999**
(21) Anmeldenummer: 95930382.7
(22) Anmeldetag: 07.09.1995
(51) Int. Cl.: C08J 11/28, C07D 251/54

(54) **VERFAHREN ZUM ABBAU VON POLYCYANURATHALTIGEN MATERIALIEN**
METHOD OF DECOMPOSING POLYCYANURATE-CONTAINING MATERIALS
PROCEDE DE DECOMPOSITION DE MATERIAUX CONTENANT DU POLYCYANURATE

(30) Priorität: 16.09.1994 DE 4432965
(43) Veröffentlichungstag der Anmeldung: 02.10.1996
(73) Patentinhaber: FRAUNHOFER-GESELLSCHAFT ZUR FÖRDERUNG DER ANGEWANDTEN FORSCHUNG E.V., 80636 München (DE)
(72) Erfinder: BAUER, Monika, D-12619 Berlin (DE); BAUER, Jörg, D-12619 Berlin (DE); GÖCKS, Karin, D-15738 Zeuthen (DE)
(86) Internationale Anmeldenummer: DE9501236
(87) Internationale Veröffentlichungsnummer: WO9608530

(56) Entgegenhaltungen:
- DE-A- 3 820 597
- SYNTHESIS, 1975, STUTTGART, DE Seiten 182 - 186 ANHE H. ET AL 'Partielle Aminolyse von 2,4,6-Triallyloxy-s-triazin. Teile 1& 2.' in der Anmeldung erwähnt

## Beschreibung

Die Erfindung betrifft ein Verfahren zum Abbau von polycyanurathaltigen Materialien, insbesondere von Polycyanuraten, Polycyanurat-Prepolymeren und polycyanurathaltigen Kunststoffen.

Polycyanurate können durch Polycyclotrimerisierung aus di- oder polyfunktionellen Cyanaten hergestellten werden. Aufgrund ihrer guten elektrischen Eigenschaften, insbesondere Isolationseigenschaften, ihrer Chemikalien- und Feuchtigkeitsbeständigkeit sowie ihrer Thermostabilität werden sie u. a. als Einschlußmittel für elektronische Bauelemente und in der Kleb-, Gieß- und Laminiertechnik eingesetzt. Zur Modifizierung ihrer Eigenschaften werden dabei die Polycyanurate mit verschiedenen Zuschlägen, beispielsweise Ruß, Silikaten oder Aluminiumoxid, versetzt und/oder mit anderen polymeren Verbindungen kombiniert.

Eine direkte Wiederverwendung von polycyanurathaltigen Materialien, sei es aus Kunststoffabfällen oder Produktionsrückständen, ist wegen der duroplastischen Eigenschaften der Polycyanurate nicht möglich. Möglich ist allenfalls die Zumischung feingemahlener Produktionsabfälle in Mengen von 15 bis 30 % zur Neuware als Füllstoff. Der übliche Weg der Entsorgung besteht deshalb in der Deponierung oder Verbrennung polycyanurathaltiger Materialien.

Verfahren zum zielgerichteten stofflichen Recycling von Kunststoffen sind bislang nur wenig erforscht. Bekannt geworden sind Thermolyse-, Pyrolyse- und Hydrierverfahren, wobei in Abhängigkeit von den Reaktionsbedingungen (und zugegebenen Katalysatoren) Synthesegase und erdölähnliche Produkte erhalten werden, die jedoch die im Ausgangsmaterial vorhandene chemische Struktur zumeist verloren haben. Diese Produkte können zwar wieder als Ausgangsstoffe in der chemischen Industrie eingesetzt werden, jedoch nur unter Einbuße eines hohen Anteils an Energie und chemischer Spezifität im Vergleich zu den ursprünglichen monomeren Ausgangsstoffen.

Für Polyester und Polyamide sind Alkoholyseverfahren bekannt geworden, in denen diese zu Monomeren oder Oligomeren abgebaut werden. Dieses Verfahren ist auf polycyanurathaltige Materialien nicht anwendbar. Ein ähnliches Verfahren ist für Polycyanurate auch nicht bekannt geworden.

Aus DE-A-3 820 597 sind eine Polyolkomponente und eine Mischung auf ihrer Grundlage zur Herstellung von Schaumstoffen bekannt.

Aus US-A-2 545 049 ist ein Verfahren zur Aminolyse von gelöstem bzw. suspendiertem Cyanursäuretriarylester bekannt geworden, wobei, in Abhängigkeit von den Reaktionsbedingungen, eine bis alle Aryloxygruppen gegen Aminogruppen ausgetauscht werden können, s. auch D. Martin et al., Journal für praktische Chemie, 323 (1981), 694. Weiterhin ist die partielle Aminolyse von geschmolzenem Cyanursäuretriallylester aus H. Ahne et al., Synthesis 182 (1975), 184 bekannt. Ausgangsprodukte und Reaktionsbedingungen dieser bekannten Reaktionen sind aber ausgesprochen speziell, so daß die allgemeine Anwendbarkeit dieser Verfahren auf polycyanuratgruppenhaltige Kunststoffe unter vollständiger Aminolyse aller Einheiten ausgeschlossen erscheint.

Insgesamt wäre es wünschenswert, ein Verfahren zu finden, mit dem polycyanurathaltige Materialien, das sind Polycyanurate, Polycyanurat-Prepolymere und polycyanurathaltige Kunststoffe, so zerlegt werden können, daß überwiegend monomere Produkte einer definierten chemischen Zusammensetzung erhalten werden. Ferner wäre es wünschenswert, wenn man die bei dieser Zerlegung anfallenden Produkte zumindest teilweise als Ausgangsstoffe für neue Polycyanurate oder andere Polymere, insbesondere solche mit verwandter Struktur, einsetzen könnte.

Der Erfindung liegt daher die Aufgabe zugrunde, ein Verfahren bereitzustellen, mit dem polycyanurathaltige Materialien so zerlegt werden können, daß definierte und wiederverwendbare Produkte unter weitgehender Beibehaltung der Struktur der im polycyanurathaltigen Material vorhandenen Komponenten erhalten werden können.

Diese Aufgabe wird mit einem Verfahren gelöst, bei dem das polycyanurathaltige Material in einem ersten Schritt in einem Lösungsmittel gelöst oder fein vermahlen wird und dadurch in eine fein verteilte Form gebracht wird und im zweiten Schritt der Aminolyse mit einem wenigstens eine reaktive NH₂-Gruppe enthaltenden Agens unterworfen wird.

Als reaktive NH₂- oder Aminogruppe im Sinne der Erfindung wird jede Aminogruppe verstanden, die in der Lage ist, eine Aminolysereaktion an Polycyanuraten einzuleiten. Dies sind insbesondere Ammoniak, Hydrazin, primäre Amine und primäre Hydrazine mit aliphatischen oder aromatischen Resten, die ihrerseits wiederum substituiert sein können. In Frage kommen daher für die Reaktion beliebige primäre Amine von gesättigten oder ungesättigten, linearen, verzweigten oder cyclischen aliphatischen oder aromatischen Kohlenwasserstoffen, die entsprechenden Hydrazine und natürlich Ammoniak und Hydrazin selbst. Die Verzweigung oder Kettenlänge des Kohlenwasserstoffrestes spielt bei der Reaktion keine Rolle, solange die Aminofunktion eine ausreichende Reaktivität gegenüber den Cyanuratgruppen aufweist.

Die zum Einsatz kommenden primären Amine können weitere Substituenten aufweisen, je nach gewünschten Substitutionsmuster der Reaktionsprodukte. Besonders vorteilhaft ist eine weitere Aminofunktion. Desweiteren können ein oder mehrere Hydroxyfunktionen vorhanden sein, ebenso beliebige andere Substituenten sofern diese nicht nachteilig in das Reaktionsgeschehen eingreifen. Dementsprechend haben die zum Einsatz kommenden Verbindung mit einer reaktiven NH₂-Gruppe die allgemeine Formel NH₂-X, worin X für Wasserstoff, OH, NH₂, R¹ oder NHR¹ steht und R¹ ein aliphatischer oder aromatischer Kohlenwasserstoffrest mit insbesondere 1 bis 20 C-Atomen ist, der substituiert sein kann. X kann insbesondere auch eine Gruppe R²-Y sein, worin R² ein zweiwertiger aliphatischer oder aromatischer Kohlenwasserstoffrest mit insbesondere 2 bis 20 C-Atomen ist und Y für einen beliebigen zweiwertigen Rest steht, insbesondere aber für NH₂, NHR¹ oder OH.

Im erfindungsgemäßen Verfahren werden die polycyanurathaltigen Augangsmaterialien durch das wenigstens eine reaktive NH₂-Gruppe enthaltende Agens zu niedermolekularen, triazinhaltigen Komponenten und di- bzw. polyfunktionellen Alkoholen abgebaut, die nach entsprechender Aufarbeitung wiederverwendet werden können.

Die erfindungsgemäß zum Einsatz kommenden polycyanurathaltigen Materialien weisen grundsätzlich das nachstehende Strukturelement I auf.

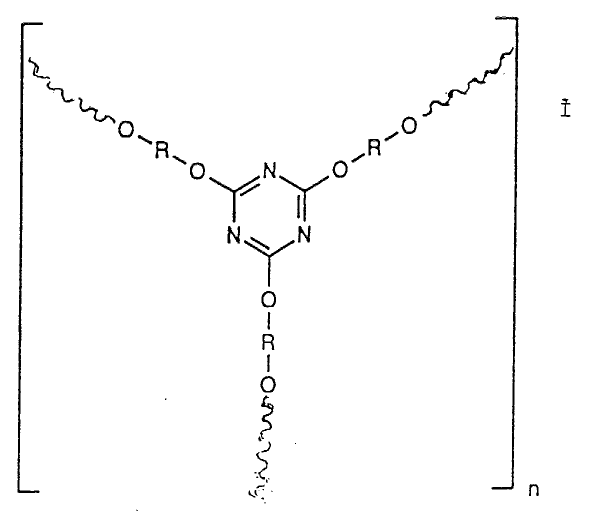

Polycyanurathaltige Prepolymere, wie sie in der Kunststofftechnik häufig zum Einsatz kommen, zeigen beispielsweise das Strukturelement der Abbildung II

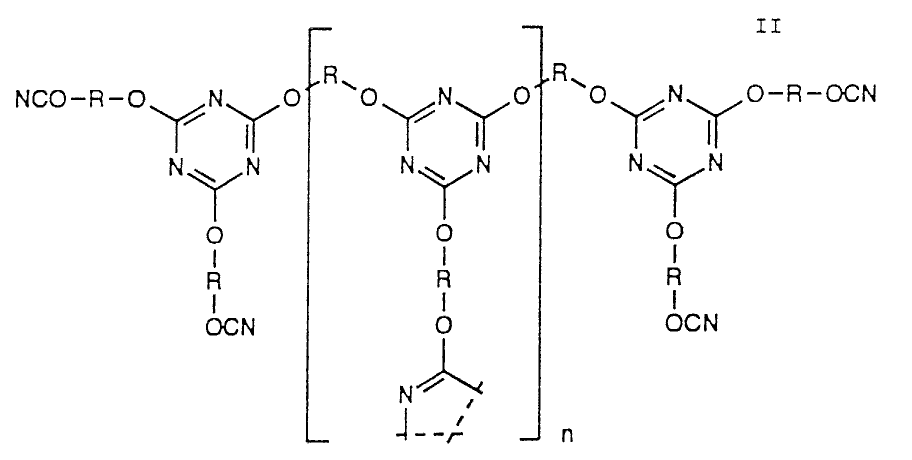

Derartige polycyanurathaltige Materialien, d. h. die fertigen Kunststoffe wie auch die Prepolymeren, können beispielsweise aus Verbindungen der Formeln III bis V erhalten werden, deren die Cyanatgruppen verbindender zweiwertiger Rest dem Rest R in den Formeln I und II entspricht. 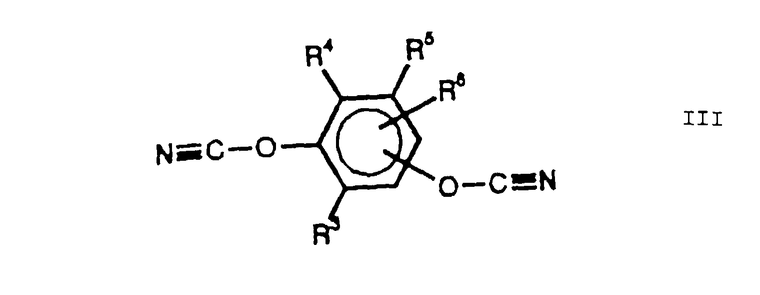 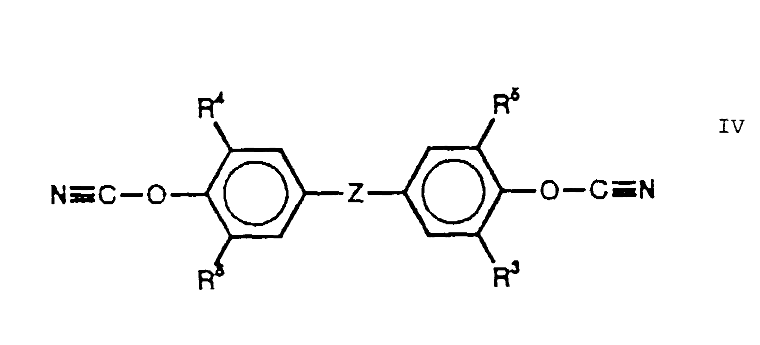 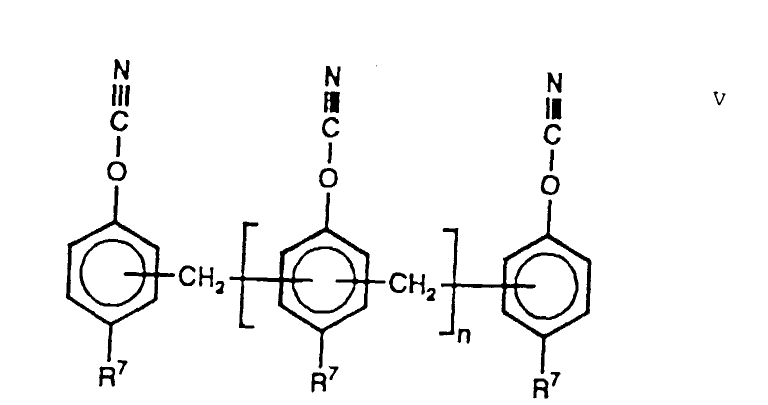 worin R³ bis R⁶ unabhängig voneinander H, C₁-C₁₀-alkyl, C₃-C₈-cycloalkyl, C₁-C₁₀-alkoxy, Halogen oder Phenyl sind, wobei Alkyl- oder Arylgruppen fluoriert oder teilfluoriert sein können; R⁷ H oder C₁-C₅-alkyl ist; Z eine chemische Bindung, SO₂, CF₂, CH₂, CH(CH₃), Isopropyl, Hexafluoroisopropyl, Alkyl, O, NR₁, N=N, CH=CH, CO-O, CH=N, CH=N-N=CH, Alkyl-O-alkyl mit C₁-C₈-alkyl, Dicyclopentadienyl, S, C(CH₃)₂ oder C(CH₃)₂-phenyl-C(CH₃)₂ darstellt; und R⁷ H, C₁-C₁₀-alkyl ist und n 0 bis 20 ist.

Weitere Di- und Polycyanate zur Herstellung von polycyanurathaltigen Kunststoffmaterialien sind bekannt; daraus abgeleitete polycyanurathaltige Materialien können ebenfalls erfindungsgemäß zerlegt werden.

Das erfindungsgemäße Verfahren führt grundsätzlich zu Produkten der Strukturformeln VI A und VI B

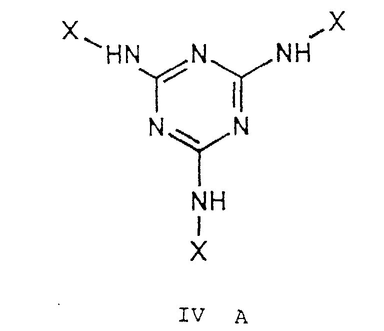

HO-R-OH VI B

worin X wie vorstehend definiert ist und R eine verbindende Gruppe ist, die sich aus den für die Herstellung verwandten Monomeren ergibt.

Für das erfindungsgemäße Verfahren ist es wesentlich, daß das polycyanurathaltige Material in fein verteilter Form vorliegt. Dies kann zum einen dadurch geschehen, daß das Material in einem geeignetem Lösungsmittel gelöst wird, beispielsweise in einem cyclischen Ether, wie THF, einem chlorierten Kohlenwasserstoff, wie Methylenchlorid, oder einem stickstoffhaltigen Lösungsmittel, beispielsweise Pyrrolidon oder NMP. Als Lösungsmittel kann auch die für die Aminolyse eingesetzte NH₂-haltige Verbindung verwandt werden, beispielsweise Ammoniak oder primäres Amin, gegebenenfalls in kondensiertem Zustand unter Druck oder aufgeschmolzen.

Im Falle von nicht löslichen Kunststoffmaterialien ist eine feine Vermahlung und Suspension im Lösungsmittel oder reaktiven Agens hilfreich. Mittlere Teilchengrößen <500 µm und insbesondere <100 um haben sich bewährt. Eine Vermahlung kann auch bei löslichen Materialien, die nicht lösliche Zuschlagsstoffe enthalten, nützlich sein.

Das erfindungsgemäße Verfahren kann bei Temperaturen von -20°C bis + 200°C, insbesondere aber bis hinauf zum Siedepunkt des verwandten Lösungsmittels oder Agens durchgeführt werden. Wird die Reaktion unter Druck mit gasförmigen Agentien, wie NH₃ oder niederen Aminen, durchgeführt, kann die Temperatur auch über den Siedepunkt des verwandten Amins hinaus gesteigert werden. Die Reaktionsgeschwindigkeit ist abhängig von der Temperatur, dem Verteilungszustand des polycyanurathaltigen Materials und dem verwandten Lösungsmittel, wobei mit fein verteilten Materialien (Lösung) bei erhöhten Temperaturen direkt im reaktivem Agens eine ausgesprochen schnelle Reaktion innerhalb von wenigen Stunden oder weniger als 1 Stunde erreicht werden kann. Durch Verwendung geeigneter Diamine oder Aminoalkohole erhält man mit dem erfindungsgemäßen Verfahren di- und/oder polyfunktionelle Amine und/oder Alkohole, die als Ausgangsstoffe für die Herstellung von Polyurethanen, Melaminharzen, Epoxidharzen, Polyestern, Polycarbonaten, etc. eingesetzt werden können. Anfallende di- bzw. polyfunktionellen Phenole können wieder in die entsprechenden di- bzw. polyfunktionellen Cyanate umgewandelt werden, welche als Ausgangsstoffe für neue polycyanurathaltige Materialien eingesetzt werden können, so daß von einem echten stofflichen Recycling gesprochen werden kann.

Die Erfindung wird durch die nachfolgenden Ausführungsbeispiele näher erläutert.

### Beispiel 1

Äquimolare Mengen von Prepolymer auf Basis DCBA mit 50% Umsatz der Cyanatgruppen und Ethylamin wurden jeweils in Tetrahydrofuran (THF) zu 10gew.-%igen Lösungen gelöst. Die Lösungen wurden vereinigt und bei 253 K 5 Tage gelagert.

Mittels Gelpermeations-Chromatographie (GPC) konnte ein vollständiger Abbau des Prepolymers nachgewiesen werden, wobei alle Abbauprodukte eine Molmasse von weniger als 1000 aufwiesen.

### Beispiel 2

Äquimolare Mengen von Prepolymer auf Basis DCBA mit 50% Umsatz der Cyanatgruppen und Dodecylamin wurden jeweils in THF gelöst, wobei 15gew.-%ige Lösungen erhalten wurden. Die Lösungen wurden vereinigt und bei Raumtemperatur einen Tag gelagert.

Mittels GPC konnte ein vollständiger Abbau des Prepolymers nachgewiesen werden, wobei alle Abbauprodukte eine Molmasse von weniger als 1000 aufwiesen. Mit Dichlormethan als Lösungsmittel konnte unter sonst gleichen Bedingungen das gleiche Ergebnis erhalten werden.

### Beispiel 3

Ausgehärtetes Polycyanurat auf Basis von Dicyanatobisphenol-A (DCBA) und Aminohexanol wurden in THF aufgeschlämmt bzw. gelöst, wobei ein 50%-iger Überschuß an Aminohexanol verwandt wurde. Anschließend wurde 66 Stunden bei 339 K unter Rückfluß gekocht.

Mittels GPC konnte ein vollständiger Abbau des Polycyanurats nachgewiesen werden, wobei alle Abbauprodukte eine Molmasse von weniger 1000 aufwiesen.

### Beispiel 4

10,8 mg pulverisiertes, ausgehärtetes Polycyanurat auf Basis von DCBA mit einer Teilchengröße <100 µm wurden mit 53,4 mg Aminohexanol versetzt und im Wasserbad 30 Minuten auf 50°C erwärmt.

Unter den genannten Bedingungen trat vollständige Aminolyse des Polycyanurats ein. Alle Abbauprodukte hatten eine Molmasse von weniger als 1000 (GPC).

### Beispiel 5

10,8 mg Granulat einer Teilchengröße <500 µm von ausgehärtetem Polycyanurat auf DCBA-Basis wurden mit 53,4 mg Aminohexanol versetzt und im Wasserbad 20 Stunden auf 65 °C erwärmt.

Ein vollständiger Abbau des Polycyanurats konnte mittels GPC nachgewiesen werden, wobei alle Abbauprodukte eine Molmasse von weniger als 1000 aufwiesen.

## Patentansprüche

1. Verfahren zum Abbau von polycyanurathaltigen Materialien, dadurch gekennzeichnet, daß das polycyanurathaltige Material in einem ersten Schritt in einem Lösungsmittel gelöst oder bis auf eine mittlere Teilchengröße < 500 µm vermahlen wird und im zweiten Schritt der Aminolyse mit einem wenigstens eine reaktive NH₂-Gruppe enthaltenden Agens unterworfen wird.

2. Verfahren nach Anspruch 1, dadurch gekennzeichnet, daß das wenigstens eine reaktive NH₂-Gruppe enthaltende Agens ein primäres aliphatisches oder aromatisches Amin oder Hydrazin ist, das weitere funktionelle Gruppen aufweisen kann.

3. Verfahren nach Anspruch 1 oder 2, dadurch gekennzeichnet, daß das wenigstens eine reaktive NH₂-Gruppe enthaltende Agens ein Aminoalkohol der Formel NH₂-Y-OH ist, worin Y ein zweiwertiger aliphatischer oder aromatischer Rest ist.

4. Verfahren nach Anspruch 1 oder 2, dadurch gekennzeichnet, daß das wenigstens eine reaktive NH₂-Gruppe enthaltende Agens ein Diamin der Formel NH₂-Y-NH₂ ist, worin Y ein zweiwertiger aliphatischer oder aromatischer Rest ist.

5. Verfahren nach einem der vorstehenden Ansprüche, dadurch gekennzeichnet, daß die Reaktion in einem cyclischen Ether, Chlorkohlenwasserstoff oder einem N-haltigen Lösungsmittel durchgeführt wird.

6. Verfahren nach Anspruch 5, dadurch gekennzeichnet, daß das Lösungsmittel THF, CH₂Cl₂ oder NMP ist.

7. Verfahren nach einem der Ansprüche 1 bis 5, dadurch gekennzeichnet, daß das wenigstens eine reaktive NH₂-Gruppe enthaltende Agens, ggfs. unter Druck in kondesiertem Zustand oder nach Aufschmelzen, als Lösungsmittel verwandt wird.

8. Verfahren nach Anspruch 7, dadurch gekennzeichnet, daß das polycyanurathaltige Material auf eine Korngröße <100 µm aufgemahlen wird.

9. Verfahren nach einem der vorstehenden Ansprüche, dadurch gekennzeichnet, daß es bei erhöhter Temperatur, durchgeführt wird.

## Claims

1. Method for the decomposition of polycyanurate materials, characterised in that the polycyanurate-containing material is dissolved in a solvent in first step or ground to an average particle size of < 500 µm and in a second step of the aminolysis is subjected to an agent containing at least one reactive NH₂- group .

2. Method according to Claim 1, characterised in that at least one agent containing a reactive NH₂- group is a primary aliphatic or aromatic amine or hydrazine which can have further functional groups.

3. Method according to Claim 1 or 2, characterised in that at least one agent containing a reactive NH₂- group is an amino alcohol of the formula NH₂-Y-OH, wherein Y is a bivalent aliphatic or aromatic radical.

4. Method according to Claim 1 or 2, characterised in that at least the agent containing a reactive NH₂- group is a diamine of the formula NH₂-Y-OH, wherein Y is a bivalent aliphatic or aromatic radical.

5. Method according to one of the previous Claims, characterised in that the reaction is performed in a cyclical ether, chlorohydrocarbon or a solvent containing N.

6. Method according to Claim 5, characterised in that the solvent is THF, CH₂CI₂ or NMP.

7. Method according to one of the Claims 1 to 5, characterised in that the agent containing at least one reactive NH₂-group is used as solvent, if necessary under pressure in a condensed state or after melting.

8. Method according to Claim 7, characterised in that the polycyanurate-containing material is ground to a particle size of < 100 µm.

9. Method according to one of the previous Claims, characterised in that it is carried out at an elevated temperature.

## Revendications

1. Procédé de dégradation de matériaux contenant du polycyanurate,
caractérisé en ce que
dans une première étape on dissout le matériau contenant du polycyanurate dans un solvant ou on le broie jusqu'à une taille particulaire moyenne inférieure à 500 µm, et dans une seconde étape on le soumet à une aminolyse avec au moins un agent contenant un groupe NH₂ réactif.

2. Procédé selon la revendication 1,
caractérisé en ce que
l'agent contenant au moins un groupe NH₂ réactif est une amine ou hydrazine aliphatique ou aromatique primaire qui peut présenter d'autres groupes fonctionnels.

3. Procédé selon la revendication 1 ou 2,
caractérisé en ce que
l'agent contenant au moins un groupe NH₂ réactif est un amino alcool de formule NH₂-Y-OH, dans laquelle Y est un radical aliphatique ou aromatique bivalent.

4. Procédé selon la revendication 1 ou 2,
caractérisé en ce que
l'agent contenant au moins un groupe NH₂ réactif est une diamine de formule NH₂-Y-NH₂, dans laquelle Y est un radical aliphatique ou aromatique bivalent.

5. Procédé selon l'une des revendications précédentes,
caractérisé en ce qu'
on conduit la réaction dans un éther cyclique, un hydrocarbure chloré ou un solvant contenant de l'azote.

6. Procédé selon la revendication 5,
caractérisé en ce que
le solvant est le THF, CH₂Cl₂ ou le NMP.

7. Procédé selon l'une des revendications 1 à 5,
caractérisé en ce que
l'agent contenant au moins un groupe NH₂ réactif est utilisé comme solvant éventuellement sous pression à l'état condensé ou après fusion.

8. Procédé selon la revendication 7,
caractérisé en ce qu'
on broie le matériau contenant du polycyanurate à une granulométrie inférieure à 100 µm.

9. Procédé selon l'une des revendications précédentes,
caractérisé en ce qu'
on opère à température augmentée.
